# EUROPEAN PATENT APPLICATION

(11) **EP 4 696 218 A1**
(43) Date of publication of application: **18.02.2026**
(21) Application number: 24194911.4
(22) Date of filing: 16.08.2024
(51) Int. Cl.: A61B 3/00, A61B 3/10

(54) **OPHTHALMIC IMAGING INSTRUMENT AND LINE SCAN ALIGNMENT CONTROL THEREIN**

(71) Applicant: Optos plc, Dunfermline, Scotland KY11 8GR (GB)
(72) Inventor: SWAN, Derek, Dunfermline, Scotland, KY11 8GR (GB)
(74) Representative: Hoffmann Eitle

(57) **Abstract**

An ophthalmic imaging instrument comprising a light source emitting a beam of light; a polygon scanning mirror comprising a plurality of reflecting facets; a driver arranged to rotate during operation said polygon scanning mirror, such that each facet reflects the beam of light at a varying angle; an optical element arranged to guide the beam reflected at a varying angle toward an eye of a subject, and a detector arranged to detect light of the reflected beam incident from a respective one of said reflecting facets and positioned in a fixed relation arrangement with said optical element.

## Description

### Technical field

The present invention relates to an ophthalmic imaging instrument and line scan alignment control therein. More particularly but not exclusively, the present invention relates to controlling the line scan alignment for improving imaging quality.

### Background

Ophthalmic imaging instruments are widely used to image a patient's eye in order to assess the health of the eye. Such systems comprise a light source and a controller that is arranged to control the light source to generate a beam of light of a target optical power. The beam of light is deflected by one or more scan relay elements so as to cover an imaging area by means of a parallel arrangement of a plurality of scanning lines. The scan relay element(s) usually comprise a polygon scanning mirror and one or more scanning galvos. The former polygon scanning mirror comprises a plurality of reflecting facets that are arranged along a circumference of a rotating body. A driver is arranged to rotate during operation the body of the polygon scanning mirror, such that each facet can reflect an incident beam of light at a varying angle. During the passing of one individual facet, the beam of light is deflected in a corresponding range of a varying angle. After completion of such a deflection cycle, the next facet substantially repeats this process and so do all other facets.

This repeated deflection by the polygon scanning mirror forms the basis for at least one scanning direction by means of providing a plurality of parallel scanning lines. A further scanning element, such as an already mentioned galvo, can then further deflect the beam in another direction so as to displace the scanning lines from each other on the target and to ultimately cover a two-dimensional scanning area. At least in some imaging modalities, the light from the scanning beam is scattered back from the target tissue which includes in the case of ophthalmic imaging primarily parts of the human eye, such as the retina. In general, the light thus travels back along the incident light path so as to be ultimately detected by an imaging detector in the form of a light sensor, converted into an intensity signal, and processed to form the image. Especially the latter signal processing is implemented by means of digital processing resources that compile individual images from corresponding sets of line scans.

As an image is compiled from a set of line scans that each run in one scanning direction, their respective alignment relative to each other may have an influence on image quality. For example, a varying scan line alignment and/or varying scan line length may lead to jitter effects in the image and, therefore, to an unsatisfyingly low image quality. As, however, the scan line lengths and their relative positions to each other may depend on the characteristics of the optical elements that deflect and guide the scanning beam of light, corresponding tolerances in the involved elements may have an impact on the mentioned image quality deterioration.

There is, therefore, a need for improved deflection control and scan line alignment in ophthalmic imaging instruments that allow for an efficient assembly of the image data at an improved image quality comprising a reduced level of image jitter, while keeping system complexity at an acceptable level.

### Summary

Problems are solved and objects are met by the subject matter of the independent claims. Further preferred embodiments are defined in the dependent claims.

According to one embodiment of the present invention, there is provided an ophthalmic imaging instrument comprising a light source emitting a beam of light; a polygon scanning mirror comprising a plurality of reflecting facets; a driver arranged to rotate during operation said polygon scanning mirror, such that each facet reflects the beam of light at a varying angle; an optical element arranged to guide the beam reflected at a varying angle toward an eye of a subject, and a detector arranged to detect light of the reflected beam incident from a respective one of said reflecting facets and positioned in a fixed relation arrangement with said optical element.

According to a further embodiment of the present invention there is provided a method for operating an ophthalmic imaging instrument comprising a light source emitting a beam of light, a polygon scanning mirror comprising a plurality of reflecting facets, an optical element, and a detector arranged to detect light of the reflected beam incident from a respective one of said reflecting facets and positioned in a fixed relation arrangement with said optical element, the method comprising rotating said polygon scanning mirror such that each facet reflects the beam of light at a varying angle guiding, by means of the optical element, the beam reflected at a varying angle toward an eye of a subject; forming an imaging signal stream by detecting light from the eye of the subject; and compiling an image of the eye of the subject from the imaging signal stream using a signal provided by the detector positioned in the fixed relation arrangement with the optical element.

### Brief Description of the Drawings

Embodiments of the present invention, which are presented for better understanding of the inventive concepts, but which are not to be seen as limiting the invention, will now be described with reference to the Figures in which:
- Figure 1: shows a schematic view of an ophthalmic imaging instrument according to a general device embodiment of the present invention;
- Figure 2: shows an optical schematic of an ophthalmic imaging instrument according to a device embodiment of the present invention;
- Figures 3A & 3B: show examples of an optical element as part of an ophthalmic imaging instrument according to a more detailed device embodiment of the present invention;
- Figure 4: shows a schematic view of an ophthalmic imaging instrument especially in the context of retrieving an imaging signal according to an embodiment of the present invention;
- Figures 5A to 5C: show schematically imaging signals as retrieved according to an embodiment of the present invention; and
- Figure 6: shows a flowchart of a general method embodiment of the present invention.

It should be understood that some of the drawings may not necessarily be shown to scale, unless otherwise indicated. In certain instances, details that are not necessary for an understanding of the disclosure or that render other details difficult to perceive may have been omitted. It should be understood, of course, that the disclosure is not necessarily limited to the particular examples or embodiments illustrated or depicted herein.

### Detailed Description

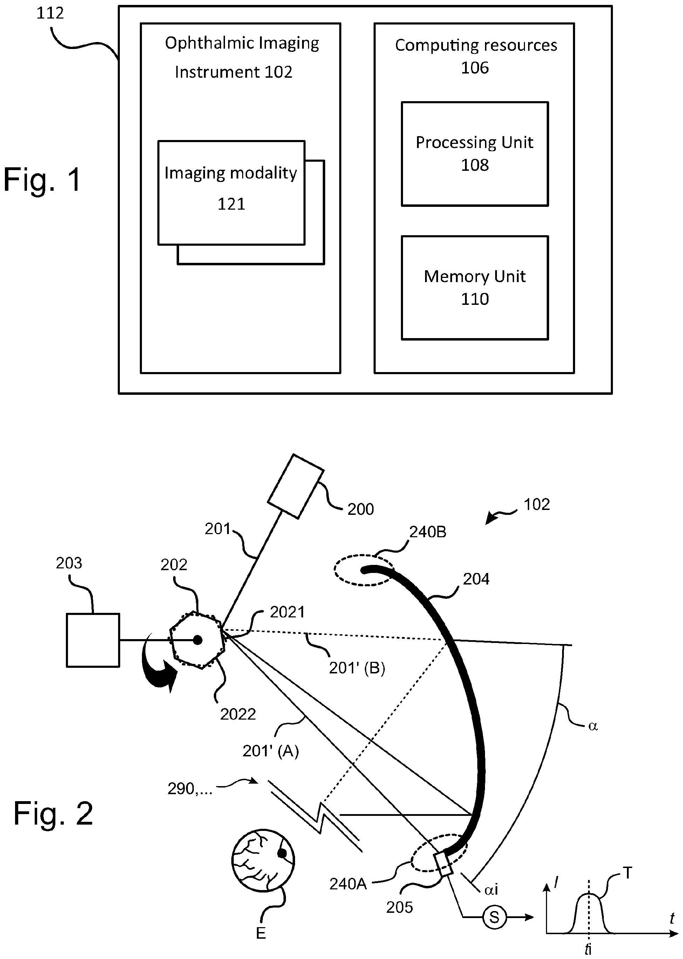

Figure 1 shows a schematic view of an ophthalmic imaging instrument according to a general device embodiment of the present invention. Specifically, there is shown an ophthalmic imaging system 112 which includes an ophthalmic imaging instrument 102 providing at least one imaging modality 121. For example, the ophthalmic imaging instrument 102 can assume the form of a scanning laser ophthalmoscope (SLO) or an optical coherence tomography (OCT) imaging instrument. The imaging modality 121 can then be understood as an operation mode in which the respective instrument is operated. Generally, the ophthalmic imaging instrument 102 can be operated in a plurality of modes, providing a plurality of corresponding imaging modalities. The ophthalmic imaging system 112 may also comprise or may have access to computing resources 106, which, in turn comprise a processing unit 108 and a memory unit 110. The components of the ophthalmic imaging system 112 including the ophthalmic imaging instrument 102 and the computing resources 106 may be housed inside a common housing so that the system 112 forms as such the ophthalmic imaging instrument, such as an ophthalmoscope. In some embodiments, the computing resources 106 may be located external to the instrument 102 within respective different housings. In some embodiments, any of the components may be located in a different housing from the rest of the components.

The computing resources 106 control the ophthalmic imaging instrument 102 to operate in the selected imaging modality 121 and the computing resources 106 have at least one processing unit 108 such as a central processing unit (CPU) and/or graphics processing unit (GPU), and a memory unit 110 that stores instructions that, when executed by the at least one processing unit 108, causes the processing unit 108 to perform one or more methods and functions described herein. In embodiments of local computing resources or local physical components of a computing device, the resources may include a processor, such as a CPU and/or GPU, a system memory, and a system bus that couples the system memory to the CPU/GPU. The system memory may include a random access memory ("RAM") and a read-only memory ("ROM"). A basic input/output ("I/O") system containing the basic routines that help to transfer information between elements within the computing device, such as during startup, is stored in the ROM. The computing resources may further include a mass storage device, which is able to store software instructions and data. The mass storage device may be connected to the CPU/GPU through a mass storage controller connected to the system bus. The mass storage device and its associated computer-readable data storage media may provide non-volatile, non-transitory storage for the computing resources 106. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device is an example of a computer-readable storage device. Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the computing device.

The computing resources 106 may operate in a networked environment using logical connections to remote network devices through a network, such as a local network, the Internet, or another type of network. The computing resources 106 may connect to the network through a network interface unit connected to the system bus. The network interface unit may also connect to other types of networks and remote computing systems. The computing resources 106 may include an input/output controller for receiving and processing inputs from a number of other devices, including a touch user interface display screen, or another type of input device. Similarly, the input/output controller may provide one or more outputs to a touch user interface display screen, a printer, or other type of output device. As mentioned above, the mass storage device and the RAM can store software instructions and data. The software instructions may include an operating system suitable for controlling the operation of the computing resources 106. The mass storage device and/or the RAM may also store software instructions, that when executed by the CPU/GPU, cause the computing resources 106 to provide the functionality discussed in this document, including the methods described herein and shown in the Figures.

In some embodiments, the imaging modality 121 may be provided as an operation mode of the already mentioned SLO, which may use confocal laser scanning microscopy for diagnostic imaging of the retina of the eye. The imaging may be two-dimensional (2D) imaging and may use a laser light beam to scan across the retina in a raster pattern to illuminate successive elements of the retina, point-by-point. The light reflected from each retinal point may be captured by a photomultiplier. The output of the photomultiplier may be recorded and displayed in a digital format. In this manner, the imaging modality in the SLO may be able to produce high-contrast, detailed images of the retina. In some embodiments, images are captured sequentially by one imaging modality and by at least a further imaging modality.

Figure 2 shows an optical schematic of an ophthalmic imaging instrument according to a device embodiment of the present invention. As shown, an ophthalmic imaging instrument 102 comprises a light source 200 emitting a beam of light 201 as a scanning beam. A polygon scanning mirror 202 acts a first scanning element (or, in other terms, a first scan relay) which comprises a plurality of reflecting facets 2021, 2022, .... A driver 203 is arranged to rotate during operation said polygon scanning mirror 202, such that each facet 2021, 2022, ... reflects the beam of light 201 at a varying angle. As illustrated, the path of the scanning beam 201 is shown in a 1D scan produced during rotation (shown by the curved arrow) of the polygon scanning mirror 202. Path "A" is an example of the scanning beam 201 reflected from a respective facet 2021 of the polygon scanning mirror 202 at a starting point during rotation, i.e. when the facet 2021 is moved into the path of the beam 201 from the light source 200. Path "B" is an example of the scanning beam 201 reflected from the respective facet 2021 of the polygon scanning mirror 202 at a later point in time further down the rotation. As the facet 2021 moves out of reach of the beam 201, the adjacent facet 2022 repeats substantially the described reflection process.

In this way, the polygon scanning mirror 202 reflects the beam 201 at a varying angle α. This angle α varies in a range that is governed by the length and rotation of the respective facet as its respective orientation relative to the incident beam 201 determines the reflection angle. As, however, the reflection process is repeated continuously as the polygon scanning mirror 202 operates and rotates, the individual facets change for each scan line. The characteristics of the polygon scanning mirror in general, and the characteristics of each facet in particular may now influence variations of the reflection behaviour of each facet amongst each iteration during which one facet 202i reflects the beam 201 into the range of the varying angle. For example, manufacturing tolerances in relation to the polygon scanning mirror may lead to variations in the facet length or other characteristics and properties that may change the reflection angle, and, with this, the respective positions of the scan lines amongst each other.

The ophthalmic imaging instrument 102 further comprises an optical element 204 arranged to guide the beam 201' reflected at a varying angle toward an eye E of a subject. Further components and optical elements 290,... may be arranged between the optical element 204 and the eye E of the subject. The optical element 204 may be or comprise a curved mirror as shown but may also take any other suitable form such as a lens or group of lenses. Embodiments considering the former curved mirror are explained in greater detail elsewhere in the present disclosure. According to this device embodiment, the ophthalmic imaging instrument 102 comprises a detector 205 which is arranged to detect the light of the reflected beam 201' which is incident from a respective one of said reflecting facets, e.g. facet 2021 as shown. The detector 205 is further positioned in a fixed relation arrangement with the optical element 204. For example, the detector 205 may be positioned in the fixed relation arrangement with the curved mirror (as a form of an optical element guiding the reflected beam toward an eye) which corresponds to a predetermined angle αi of the range of the varying angle. For example, the predetermined angle αi may correspond to starting angle at which a start of a reflection cycle, and, with this, an individual scan line is assumed. Generally, the detector may act as a line start sensor 205 and may be positioned proximate to the optical element 204, for example at/near an edge or in a periphery region 240A, 240B of the optical element 204.

The detector 205 may be arranged to generate and output a signal S, for example, in the form of a line-start pulse signal. In an example, the detector 205 provides a current and/or voltage response that is relative to the intensity of incident light. Therefore, the signal S may comprise a pulse-like feature T in an intensity-versus-time profile *I*(t) as shown in the inset in figure 2. This feature T in the intensity *I* as part of the signal S may indicate a time ti when the beam 201' is at an angle αi, which, in turn, can be taken as an indication that the beam is at a starting point of a scan line. This signal S can be employed to align and/or synchronize the scan lines amongst each other considering, in particular, also scan lines that are generated by different facets of the polygon scanning mirror 202 that may have different characteristics, especially regarding a facet length. The present embodiments allow for an efficient synchronizing of the scan lines and the resulting imaging signals as the detector may provide an output that at least defines or indicates one common point for each scan line regardless of the facet that is acting as reflector and regardless of that individual facet's properties and characteristics.

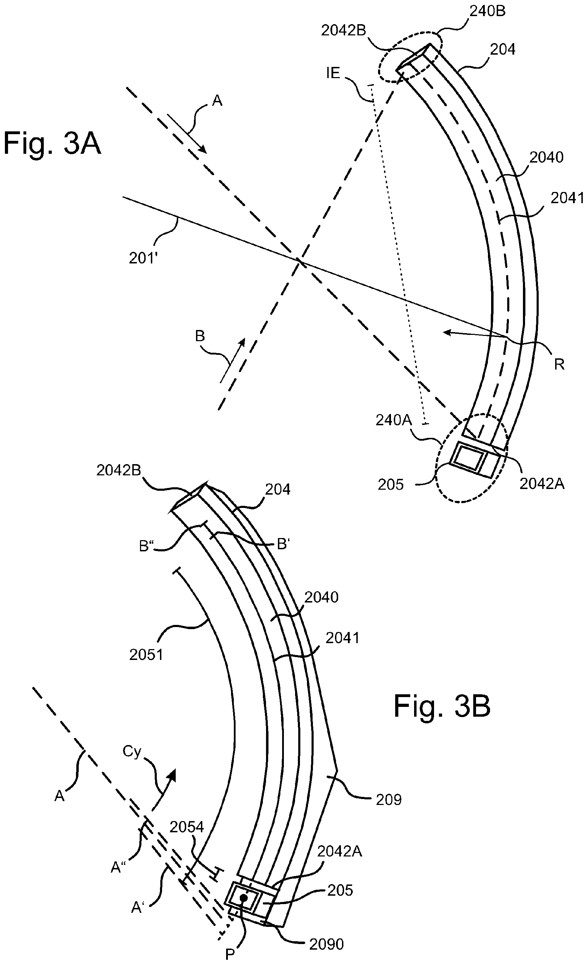

Figure 3A shows an example of an optical element as part of an ophthalmic imaging instrument according to a more detailed device embodiment of the present invention. As described elsewhere in the present disclosure, a light source provides a beam of light that is directed at a polygon scanning mirror with a plurality of reflecting facets. As a driver rotates the polygon scanning mirror, each facet reflects the beam of light at a varying angle into an optical element that further guides the beam reflected at a varying angle toward an eye of a subject. In general, an optical element according to the embodiments should be able to direct a beam of light that is incident from different directions further toward the imaging target. For this purpose, the optical element may provide for a finite input extension for capturing the beam of light incident from different directions as a result of being reflected at a varying angle at the upstream optical element.

Accordingly, Fig. 3A shows an example of such an optical element in the form of a curved mirror 204 in an elongated form such as to be able to capture a beam of light incident from different directions, such as a first direction A and a second direction B. The optical element thus comprises a curved mirror which is arranged to guide the beam incident from a respective one of said reflecting facets toward the eye of the subject by further reflecting the beam along a reflection line on the curved mirror. For this purpose, the curved mirror 204 provides for an input extension IE that may cover a range of different directions and angles of the beam of light 201'. Therefore, the curved mirror 204 provides for an extended reflection area 2040 which, in turn, accommodates a reflection line 2041 along which the incident beam of light 201' is reflected as the angle of incidence changes. Assuming a continuous rotation of the polygon scanning mirror as the upstream optical element, the reflection point R will move along the reflection line 2041 accommodated by the extended reflection area 2040. As the repeated reflection by the polygon scanning mirror forms one scan direction, the reflection line 2041 provides for one scan line of the beam of light. If the incident angle varies within a greater range than the curved mirror 204 can cover, it is then the curved mirror 204 that effectively governs the length of a scan line. This may be for example the case if the extended reflection area 2040 is too short by not covering (and reflecting) the incident beam of light at one or more ends 2042A, 2042B. Alternatively, the length of a scan line can of course also be governed by any other optical element, for example if the curved mirror 204 is arranged to cover a full range of varying incident angles and directions.

Figure 3B shows an example of an optical element as part of an ophthalmic imaging instrument according to a more detailed device embodiment of the present invention, and there is again shown an optical element in the form of a curved mirror 204 as explained amongst others in conjunction with Figure 3A (same reference signs thus denote same elements and functionalities). Specifically, this embodiment considers an arrangement of a detector so that it detects at least at one point in a reflection cycle light of the reflected beam incident from all facets of the polygon scanning mirror. A reflection cycle may be defined by reflecting the beam of light by one single facet of the polygon scanning mirror as the latter rotates.

For example, the fixed relation arrangement of the detector and optical element may be such that the beam reflected at a varying angle hits a detection point of the detector for all facets of the polygon scanning mirror. In this embodiment, the curved mirror 204 is shown together with a detector 205 that is arranged to detect light of the incident beam as being reflected from a respective one of reflecting facets of an upstream polygon scanning mirror. The detector 205 is positioned in a fixed relation arrangement with the curved mirror 204 so that the position of the detector 205 can be assumed to have a well-defined and constant relationship with the positions in a scan line. For example, a line 2051 may represent the line in a (curved) plane of the curved mirror 204 which the incident beam of light 201' intersects as the upstream polygon scanning mirror rotates. A start direction A may mark a beginning of this line 2051, and an end direction B may mark an end of this line 2051. As shown, the incident beam of light 201' may start at an angle at which it neither hits the detector 205 nor reflecting surface of the curved mirror 204. In this way, the incident beam of light 201' may always hit the detector 205, so that detection of light at this detector 205 can provide reliable information for a starting point of a scan line.

Specifically, the start direction A may vary due to properties of any upstream optical element. For example, the facets of an upstream polygon scanning mirror may have varying characteristics, such as a varying length, due to manufacturing tolerances. As a result, the start direction may vary such that for a longer facet length the direction is A' and for a shorter facet length, the direction is A". Preferably, the detector 205 is positioned in a fixed relation arrangement with the curved mirror 204 and other elements of the ophthalmic imaging instrument so that all occurring starting directions ranging from A' as a respective minimum to A" as a respective maximum of a variation lie before the position of the sensor 205. A maximum range for the incident direction may thus be as shown with the range 2051, wherein a lower start point may vary as described in conjunctions with directions A, A' and A" and an upper stop point may vary as well (see B' and B"). However, the latter may be of lower relevance only, since the embodiments of the present invention allow for determining a reproducible position of at least one point, which, in turn, may be sufficient to position the scan lines amongst each other.

Specifically, the incident beam of light 201' may start its cycle with a direction ranging between directions A' and A" and proceed the cycle as indicated by the arrow Cy. The start of the cycle thus always lies before the position and a detection point P of the detector 205 along a reflection line 2041. It is noted that this line may be extended virtually beyond the end 2042A of the curved mirror 204 as in this region of course no reflection by the mirror can occur. In this way, the detector 205 is positioned in a fixed arrangement with the curved mirror 204 so that, when a reflection cycle Cy starts for each facet of the polygon scanning mirror, the incident beam 201' hits first the detector 205 (e.g. specifically a detection point P thereof) and then a reflecting area 2040 of the curved mirror 204. A dead band 2054 may exist between the start points or the detection point P and the beginning of reflection at the end 2042, which would lead to a corresponding dead band in the respective image signal. Such a dead band may, however, be acceptable, as the fixed relation arrangement of the detector with the optical element in the form of the curved mirror 204 can be taken as a basis for filtering out the respective data as explained elsewhere in the present disclosure.

Generally, an ophthalmic imaging instrument may further comprise a carrier on which the optical element and the detector are mounted. As an example, and as shown in Figure 3B, such a carrier can be in the form of a mirror carrier 209 which may comprise the features for mounting and stabilizing the optical element in the form of a curved mirror, which may comprise features for suppressing vibrations and or temperature-induced deflections. For example, the carrier may provide for a fitting shape that holds and stabilizes the curved mirror for a substantial part of its respective extension. In the shown example, the carrier 206 provides for mounting plateau 2090 for the detector 205. For example, the shape fittingly accommodating a curved mirror can be extended continuously into a planar plateau. The carrier may be manufactured of one piece, such that it is formed to be a single continuous body by means of, for example, die casting.

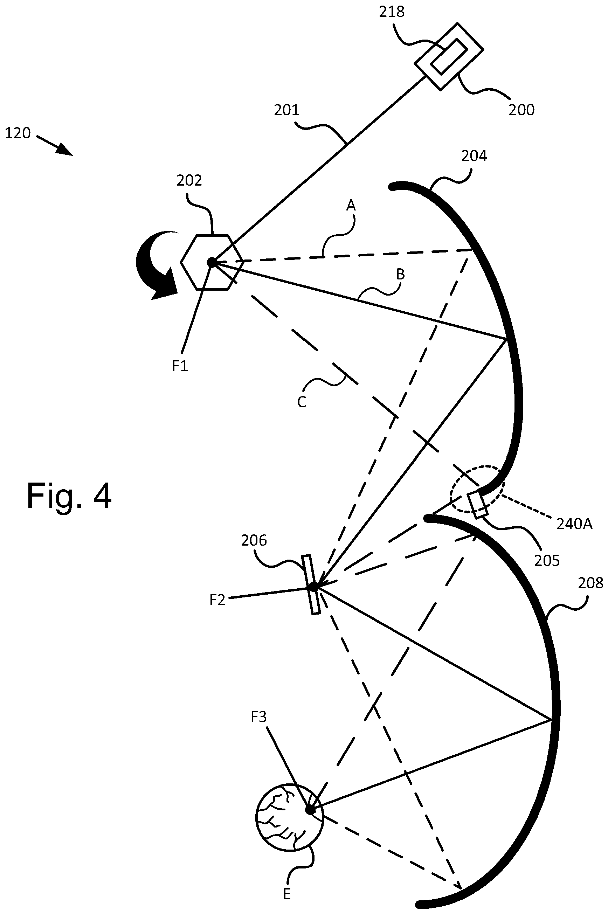

Figure 4 shows a schematic view of an ophthalmic imaging instrument especially in the context of retrieving an imaging signal according to an embodiment of the present invention and there are again shown elements as explained amongst others in conjunction with Figures 3A and 3B (same reference signs thus denote same elements and functionalities). The ophthalmic imaging instrument 120 is described in the context of an imaging modality in which a beam of light 201 is generated by the light source 200 and guided toward a patient's eye E, reflected at tissue of the same and guided back to a detector 218. As part of, for example, an SLO, the instrument 120 includes the light source 200 emitting beam of light, that is, a scanning beam 201, and a plurality of scan relay elements. The scan relay elements include a first scanning element in the form of a polygon scanning mirror 202, a second scanning element 206, and optical elements that are positioned and configured to direct the scanning beam 201. In some embodiments, the optical elements may include the optical element 204 which may be a scan compensation element such as a curved mirror or slit mirror, and a second optical element 208 which may be a scan transfer element such as a main mirror.

The optical elements 204 and 208 are positioned and configured to direct the scanning beam 201. The second scanning element 206 may be or include an oscillating plane scanning mirror or a planar scanning mirror coupled to a galvanometer motor. The optical element 204 may be a curved mirror as an ellipsoidal mirror. The second optical element 208 may be an aspherical mirror. It is to be appreciated that the first and second optical elements may have an alternative form. The scanning elements 202 and 206 may be referred to as a scanning device or a plurality of individual scanning devices. It is to be understood that the shown functionalities are just an example of a configuration that can be used with the embodiments described herein.

A detector 205, for example in the form and with the functions of a line start sensor is disposed in or along an optical path of the scanning beam 201, and is positioned in a fixed relation arrangement with the optical element 204. In some embodiments, the detector 205 may be positioned proximate to the optical element 204, for example at/near an edge or near the periphery region 240A of the optical element 204. The detector 205 may be configured to generate and output a signal, for example a line-start pulse signal, as the scanning beam 201 crosses a detection point of detector 205. For example, such a detection point may be defined by an aperture or the opening of an optical sensor providing a well-defined position at which incident light is detected. In some examples, the detector 218 may be a separate component or device that is operatively coupled with the light source 200 such that the detector 218 and the light source 200 may be positioned at the same location or adjacent to one another. In some examples, the light source 200 may be implemented with the detector 218 as a single device, or devices within a common housing, such that in addition to emitting the scanning beam 201, the device may also be capable of detecting or receiving light, such as the light reflected back from the first scanning element 202 or from the optical element 204.

In an exemplary SLO which operates in an exemplary imaging modality, the scanning beam 201 may be a laser of suitable wavelength as used in SLO applications. If other imaging modalities are included, the scanning beam 201 may be a collimated light which includes the laser for SLO applications and a superluminescent diode (SLD) for other applications, for example. It should be appreciated that any suitable source of collimated light may be used, such as a single frequency laser diode, vertical-cavity surface-emitting laser, wavelength swept laser source, pulsed laser source, or other source that has enough intensity and to be well collimated and produce adequate retinal illumination. In such applications, the SLD may be used due to the short coherence lengths required to discriminate the retinal layers from the resultant interferometric data. The SLD may be free space or fiber coupled into standard or polarization maintaining fiber to the scan system. A swept source laser may also be used in other applications, whereby the wavelength of the source is tuned over a given range.

In some embodiments, one or more of the scanning elements may include one or more of: an oscillating plane mirror, a galvanometer mirror, a MEMS mirror, a rotating mirror, prism or polygon scanner, and/or a resonant mirror, for example. In some embodiments, each of the first scanning element 202 or the second scanning element 206 may be a single element or an arrangement of two or more elements as suitable to provide a scan at a respective focal point F1 or F2, as shown, at which the scanning element is disposed. The focal points F1 and F2 are the foci of the optical element 204, and the focal points F2 and F3 are the foci of the optical element 208. The first scanning element 202 is positioned at focal point F1, the second scanning element 206 is positioned at focal point F2, and the eye 210 is positioned at focal point F3 (also referred to as a virtual scanning point). The resulting scan may be a 2D scan or scan pattern of the scanning beam 201 as the light sweeps through the virtual scanning point (e.g., through F3) inside the eye 210.

In some embodiments, the first scanning element 202 provides either a vertical, horizontal, or patterned scan which is incident on the optical element 204, to a point on the second scanning element 206 via the optical element 204. The scans may be one-dimensional (1D) or two-dimensional (2D) light scans, for example. The axes of the first scanning element 202 and the second scanning element 206 may be arranged to create a 2D light scan, such as in the form of a raster scan pattern of the scanning beam 201. The alignment of the first and second scanning elements 202, 206 may be orthogonal, substantially orthogonal, or arranged to generate an arbitrary scan geometry about the optical elements 204 and 208.

In some embodiments, the second scanning element 206 provides a plurality of scans, for example 1D or 2D light scans, which may comprise horizontal scans, vertical scans, or arbitrary patterns of the scanning beam 201. The scans provided by the first scanning element 202 and the scans provided by the second scanning element 206 are different from each other, for example with respect to the orientation of the scans. In some examples, one of the scanning elements may provide vertical scans of the retina, and the other scanning element may provide horizontal scans of the retina. The scanning beam 201 is directed towards a patient's eye E via the scanning elements 202 and 206, and the optical elements 204 and 208, such that an ultra-wide field scan angle is achieved at the pupil plane of the eye E.

A "wide field" scanning refers to a scan angle in excess of 50 degrees in one or two dimensions. An "ultra-wide field" scanning refers to a scan covering substantially the entire retina of the eye E. In some examples, the plurality of line scans may be generated by scanning the retina along a first direction using the first scanning element 202, and positions of the plurality of line scans are varied along a second direction using the second scanning element 206, where the second direction is orthogonal to the first direction. As illustrated in Figure 4, the path of the scanning beam 201 is shown in a 1D scan produced by one oscillation or rotation (shown by the curved arrow) of the first scanning element 202. Path "A" is an example of the scanning beam 201 reflected from the polygon scanning mirror at one orientation of a reflecting facet during rotation, whereas paths "B" and "C" are examples of the scanning beam 201 reflected at other orientations of the facet during rotation.

The components of the ophthalmic imaging instrument 120 may be arranged such that the rotational axis of the first scanning element 202 is substantially parallel to a line joining the two foci of the optical element 208 (i.e., F2 and F3) such that the scanning beam 201 is scanned across the secondary axis of the optical element 204. Furthermore, the first scanning element 202 may produce a 1D or 2D scan which is incident on the optical element 204. The optical element 204 may also therefore produce a 1D or 2D scan. The components of the ophthalmic imaging instrument 102 may be arranged such that the line joining the two foci of the optical element 208 (i.e., F2 and F3) lies substantially on a plane defined by the scan (e.g., 1D vertical scan) produced by the optical element 204.

The first and second scanning elements 202 and 206 may thus together create a light scan, for example a 2D scan, in the form of a raster scan pattern from a single point in space at or near the focal point F3 at or in the eye E of the patient. The first and second scanning elements may have operating parameters which include the amplitude of the oscillation and the rotational offset of the oscillation. The operating parameters also include the velocity of oscillation. Both of these operating parameters may be selected to control the direction and pattern of the light scan from the apparent point source. In some examples, the first and second scanning elements may be housed in a rotation mount (not shown) that can adjust the centering (or eccentricity) of the scanning beam 201 on the retina of the eye E, which provides the ability to "move" the imaging field across the retina.

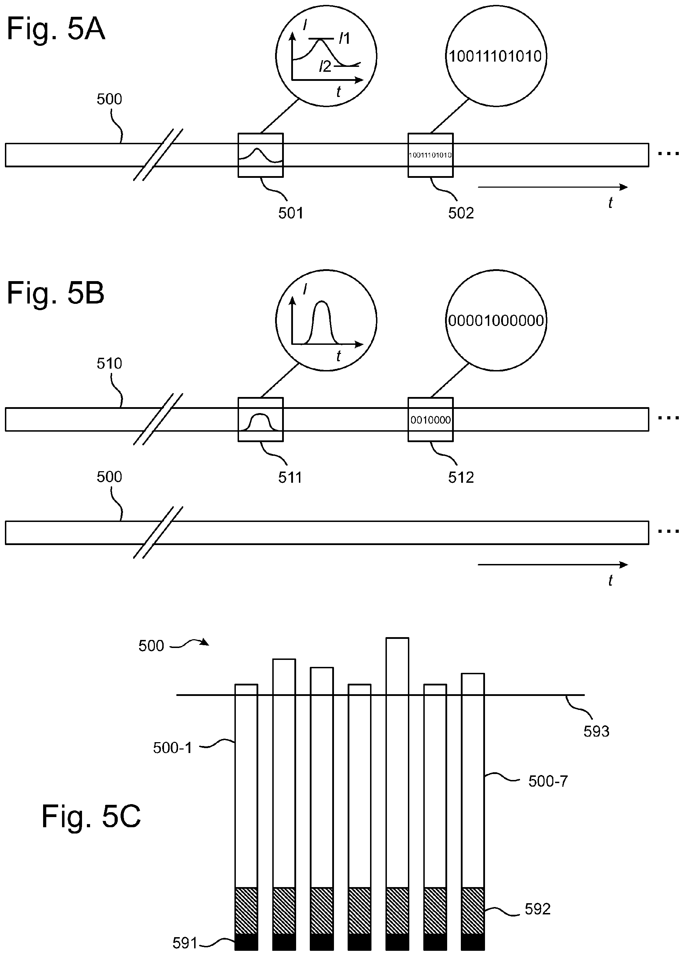

Figures 5A to 5C show schematically imaging signals as retrieved according to an embodiment of the present invention. In Figure 5A there is shown a schematic representation of a signal stream 500 representing the output of an imaging detector as for example detector 218 as explained in conjunction with Figure 4. Specifically, the signal stream 500 may represent the detector output versus time t in the form of an intensity signal. For example, a first detected light intensity may be represented as an analogue signal I1, and a second detected light intensity may be represented as an analogue signal I2 (as shown for the case of an analogue signal for a signal section 501.

Alternatively, in a digital implementation, the signal stream 500 may represent a stream of numerical digital values, wherein a set of information bits may represent one intensity value. For example, the binary number 00001111 may represent an intensity value of decimal 15, which, in turn, may correspond to a known intensity value or a corresponding detector output voltage or current. It is noted that the analogue form 501 and the digital form 502 may coexist with an ophthalmic imaging system according to the embodiments of the present invention. Specifically, the signal stream may be initially provided as an analogue signal such as 501 as an immediate output of the detector 218 and then be analogue-to-digital converted, ADC, to be in the form as shown in the digital form 502. The latter may be suitable for later data processing by means of digital computing resources so as to ultimately compile the respective image data to be displayed to a user of the instrument.

In Figure 5B there is shown the schematic representation of the signal stream 500 as explained before but in conjunction with a schematic representation of a signal output by a detector which is arranged to detect light of the reflected beam incident from a reflecting facet of a polygon scanning mirror and which is positioned in a fixed relation arrangement with the optical element that guides the beam of light toward the eye of a subject. For example, signal 510 may represent the output of a detector 205 as explained in conjunction with any embodiment of the present disclosure. The signal stream 510 may represent the intensity versus time t as an output of detector 205. For example, an analogue signal may comprise a peak amplitude as indicating the passing of the beam of light incident from the polygon scanning mirror at a detection point of the detector (see e.g. detection point P of detector 205 in Figure 3B). This is shown for the case of an analogue signal as a peak amplitude in the signal section 511.

Alternatively, in a digital implementation, the signal stream 500 may represent a stream of numerical digital values, wherein a set of information bits may represent the intensity value detected by detector 205, in which, for example, one or more bits "1" may indicate the passing of the beam of light at the detection point of the detector. It is noted that the analogue form 511 and the digital form 512 may coexist with an ophthalmic imaging system according to the embodiments of the present invention. Specifically, the signal stream may be initially provided as an analogue signal such as 511 as an immediate output of the detector 205 and then be analogue-to-digital converted, ADC, to be in the form as shown in the digital form 512. The latter may be suitable for later data processing by means of digital computing resources so as to ultimately compile the respective image data to be displayed to a user of the instrument. For this purpose, the signal streams 500 and 510 are processed in synchronization to each other as explained below.

Figure 5C shows schematically the processing of the imaging signal stream 500 as originating, for example, from the imaging sensor 218. Specifically, the stream 500 is processed into individual scan lines represented by sections 500-1 to 500-7 of the stream 500. It is noted, that a section 500-(i+1) may start in the original stream 500 after a preceding section 500-i has ended. In the embodiments of the present invention the output of the detector 205 may be employed to section the signal stream 500 into individual scan lines. Specifically, the passing of the detector 205 by the beam of light 201' incident from the polygon scanning mirror can be taken as start positions 591 for the individual sections 500-1, .... With this, any varying lengths, e.g. caused by varying characteristics of the individual reflecting facets of the polygon scanning mirror, may be compensated for, as each scan line starts at the same well-defined position and any variations toward the end of a scan line can be ignored or cut off after a position indicated by the line 593. Each section may also comprise data representing a dead band 592, as this may be caused by a finite distance between the detection point of a detector 205 and the beginning of a reflecting surface of the optical element guiding the beam reflected by the polygon scanning mirror at a varying angle toward an eye of a subject. The detector 205 may be configured to generate and output a signal for example in the form of a line-start pulse signal. It is noted that the start of a scan line is just an example, as the alignment of a plurality of scan lines may also be obtained by means of knowing a common line-stop point or any other well-defined intermediate point. This signal may in any way form a synchronization signal for image data processing and/or acquisition.

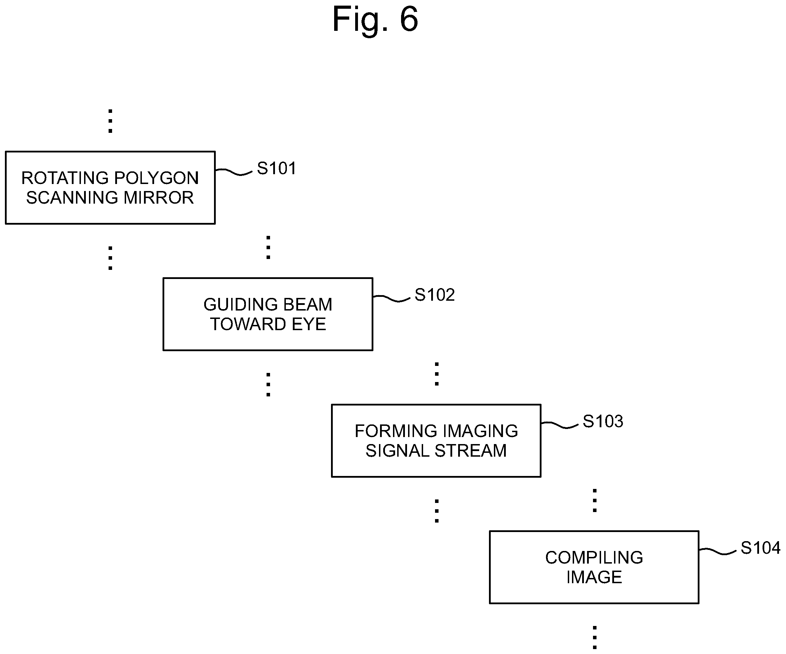

Figure 6 shows a flowchart of a general method embodiment of the present invention. Specifically, the method embodiments relate to operating an ophthalmic imaging instrument that comprises a light source, a polygon scanning mirror, an optical element, and a detector arranged to detect light of the reflected beam incident from a facet of the polygon scanning mirror and positioned in a fixed relation arrangement with said optical element. For example, the methods may be suitable for operating an ophthalmic imaging instrument as the instrument 102 or 120 as disclosed and described in conjunctions with embodiments of the present disclosure. The method comprises a step S101 of rotating the polygon scanning mirror such that each facet reflects the beam of light at a varying angle, a step S102 of guiding, by means of the optical element, the beam reflected at a varying angle toward an eye of a subject, a step S103 of forming an imaging signal stream by detecting light from the eye of the subject, and a step S104 of compiling an image of the eye of the subject from the imaging signal stream using a signal provided by the detector positioned in the fixed relation arrangement with the optical element. For example, the imaging signal stream formed in step S103 may be processed using a signal from a detector 205 as described in conjunctions with

Figures 5A to 5C so as to compile the image in step S104. It is noted that the steps S101 to S104 can be performed concurrently and/or at least in part sequentially as is most suitable for the chosen implementation.

In a further method embodiment, the step S104 of compiling an image may comprise detecting a start of a scan line based on the signal provided by the detector. Further, the step S104 of compiling an image may comprise aligning adjacent scan lines based on a detected start of a respective scan line. For example, a detector may be employed to section a signal stream into individual scan lines, wherein the passing of the detector by the beam of light incident from the polygon scanning mirror can be taken for determining a start of a scan line (see e.g. positions 591 for the individual scan lines 500-1, ... in Figure 5C). With this, adjacent scan lines can be aligned under consideration of the respective start points as determined so as to improve image quality as even varying lengths, e.g. caused by varying characteristics of the individual reflecting elements, are compensated for.

The forgoing has presented embodiments and details thereof as part of the present invention which can provide one or more advantages by improving deflection control and scan line alignment in ophthalmic imaging instruments that allow for an efficient assembly of the image data at an improved image quality comprising a reduced level of image jitter, while keeping system complexity at an acceptable level. While various embodiments of the present disclosure have been described above, it should be understood that they have been presented by way of example, not limitation. It will be apparent to persons skilled in the relevant art(s) that various changes in form and detail can be made therein. Thus, the above-described exemplary embodiments are not limiting.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

## Claims

1. An ophthalmic imaging instrument (102) comprising:
a light source (200) emitting a beam (201) of light;
a polygon scanning mirror (202) comprising a plurality of reflecting facets (2021, 2022,...);
a driver (203) arranged to rotate during operation said polygon scanning mirror (202),
such that each facet (2021, 2022,...) reflects the beam of light at a varying angle (α);
an optical element (204) arranged to guide the beam reflected at a varying angle toward an eye (E) of a subject, and
a detector (205) arranged to detect light of the reflected beam incident from a respective one of said reflecting facets (2021, 2022,...) and positioned in a fixed relation arrangement with said optical element (204).

2. The ophthalmic imaging instrument (102) according to claim 1, wherein the detector (205) is positioned in the fixed relation arrangement with said optical element (204) corresponding to a predetermined angle of a range of said varying angle (α).

3. The ophthalmic imaging instrument (102) according to claim 2, wherein the predetermined angle corresponds to a start of a scan line.

4. The ophthalmic imaging instrument (102) according to any one of claims 1 to 3, wherein the detector (205) is arranged to detect light of the reflected beam incident from all facets of the polygon scanning mirror (202).

5. The ophthalmic imaging instrument (102) according to claim 4, wherein the fixed relation arrangement of the detector (205) and optical element (204) is such that the beam reflected at a varying angle (α) hits a detection point (P) of the detector (205) for all facets (2021, 2022,...) of the polygon scanning mirror (202).

6. The ophthalmic imaging instrument (102) according to any one of claims 1 to 5, wherein the optical element (204) comprises a curved mirror, arranged to guide the beam incident from a respective one of said reflecting facets (2021, 2022,...) toward the eye € of the subject by further reflecting the beam along a reflection line on the curved mirror.

7. The ophthalmic imaging instrument (102) according to claim 6, wherein the detector (205) is positioned in a fixed arrangement with the curved mirror so that, when a reflection cycle starts for each facet of the polygon scanning mirror (202), the incident beam hits first the detector (205) and then a reflecting area of the curved mirror.

8. The ophthalmic imaging instrument (102) according to any one of claim 1 to 7, further comprising a carrier (209) on which the optical element (204) and the detector (205) are mounted.

9. The ophthalmic imaging instrument (102) according to claim 8, wherein the optical element (204) arranged to guide the beam (201) toward an eye (E) of a subject is formed of a curved mirror, wherein the carrier (209) comprises a fitting shape for accommodating the curved mirror and a mounting plateau (2090) arranged for holding the detector (205).

10. The ophthalmic imaging instrument (102) according to any one of claims 1 to 9, wherein the detector (205) is arranged to provide a signal being used for aligning scan lines during imaging processing.

11. The ophthalmic imaging instrument (102) according to any one of claims 1 to 10, further comprising processing resources arranged to compile an image of the eye (E) of the subject using a signal provided by the detector (205) positioned in the fixed relation arrangement with the optical element (204).

12. The ophthalmic imaging instrument (102) according to claim 11, wherein the processing resources are arranged to use the signal provided by the detector (205) as a synchronization base for forming sections representing scan lines in one direction of the image.

13. A method for operating an ophthalmic imaging instrument comprising a light source emitting a beam of light, a polygon scanning mirror comprising a plurality of reflecting facets, an optical element, and a detector arranged to detect light of the reflected beam incident from a respective one of said reflecting facets and positioned in a fixed relation arrangement with said optical element, the method comprising
- rotating (S101) said polygon scanning mirror such that each facet reflects the beam of light at a varying angle
- Guiding (S102), by means of the optical element, the beam reflected at a varying angle toward an eye of a subject;
- forming (S103) an imaging signal stream by detecting light from the eye of the subject;
- compiling (S104) an image of the eye of the subject from the imaging signal stream using a signal provided by the detector positioned in the fixed relation arrangement with the optical element.

14. The method of claim 13, wherein the step (S104) of compiling an image further comprises detecting a start of a scan line based on the signal provided by the detector.

15. The method of claim 13 or 14, wherein the step (S104) of compiling an image further comprises aligning adjacent scan lines based on a detected start of a respective scan line.
